# FASCICULE DE BREVET EUROPEEN

(11) **EP 4 444 192 B1**
(45) Date de publication et mention de la délivrance du brevet: **05.03.2025**
(21) Numéro de dépôt: 22801860.2
(22) Date de dépôt: 18.10.2022
(51) Int. Cl.: A61B 17/064, A61B 17/068, A61B 17/10, A61B 17/04, A61B 17/062, A61B 17/29, A61B 17/30, A61F 5/00

(54) **DISPOSITIF DE PLICATURE GASTRIQUE PAR VOIE ENDOSCOPIQUE**
ENDOSKOPISCHE MAGENPLIKATIONSVORRICHTUNG
ENDOSCOPIC GASTRIC PLICATION DEVICE

(30) Priorité: 12.01.2022 FR 2200220
(43) Date de publication de la demande: 16.10.2024
(73) Titulaire: Endoply, 69140 Rillieux-La-Pape (FR)
(72) Inventeur: PAIN, Bernard, 43120 Monistrol-sur-Loire (FR); DARGENT, Jérôme, 69002 Lyon (FR)
(74) Mandataire: Cabinet Laurent & Charras
(86) Numéro de dépôt international: PCT/FR2022/051964
(87) Numéro de publication internationale: WO 2023/135370

(56) Documents cités:
- EP-A1- 2 189 120
- US-A1- 2013 153 642
- US-A1- 2013 338 680
- US-A1- 2015 126 983

## Description

### Domaine technique

La présente invention se rapporte au domaine technique des dispositifs chirurgicaux utilisés pour le traitement de l'obésité, et concerne plus particulièrement un dispositif de plicature gastrique par voie endoscopique.

### Art antérieur

Dans le domaine des dispositifs chirurgicaux utilisés pour le traitement de l'obésité, il est connu le document US20150126983 qui décrit un dispositif de plicature gastrique par voie endoscopique comprenant un ensemble apte à coopérer avec une poignée assujettie à des moyens de commande.

Les moyens de commande sont conçus pour actionner l'ensemble pour la mise en place et la fixation d'un fil de suture autour d'un pli formé dans une paroi gastrique, en vue de créer une restriction du fundus et de l'antre gastrique.

Dans l'art antérieur, une aiguille hélicoïdale est destinée à venir s'ancrer dans la paroi de l'estomac et à être tirée en arrière pour former un pli de la paroi gastrique.

Ensuite, une aiguille traversée par le fil de suture vient faire un point de suture au travers du pli. La réalisation de deux points de sutures éloignés et reliés entre eux permet, après avoir tendu le fil, de rapprocher lesdits deux points de sutures pour plier davantage la paroi gastrique et en diminuer son volume.

L'inconvénient de ce type de dispositif, outre sa complexité et son coût de revient, est en premier lieu l'utilisation d'un fil de suture qui peut potentiellement provoquer une déchirure muqueuse et/ou séreuse plus ou moins importante, notamment sur le long terme. La tenue dans le temps d'une suture est donc limitée.

L'utilisation de l'aiguille hélicoïdale pour former le premier pli présente une efficacité aléatoire et peut également provoquer une déchirure muqueuse et/ou séreuse, mais surtout, l'aiguille hélicoïdale reste souvent coincée dans les tissus et provoque une hémorragie qui gêne la vision endoscopique.

L'opération avec ce type de dispositif est également complexe et longue puisqu'il convient de tracer deux lignes sensiblement parallèles dans l'estomac, qui doivent servir de guide pour le positionnement des points de sutures, qui devront être ensuite tendu à la manière de lacets de chaussure pour refermer et restreindre une partie de l'estomac. Au surplus, l'étanchéité de la partie restreinte de l'estomac n'est pas optimale.

D'une manière générale, les dispositifs mettant en œuvre un fil de suture allongent de manière considérable la durée de l'intervention chirurgicale. En effet, pour chaque point de suture, il est nécessaire de préparer et présenter le fil, de l'introduire dans l'endoscope, de le positionner à l'extrémité de l'endoscope, de saisir la paroi gastrique avec un instrument séparé, de faire un bouclage du fil, de verrouiller le fil après application d'une tension. Toutes ces opérations sont réalisées de l'extérieur par un assistant. En dernier lieu, il convient de sectionner le fil et de retirer le dispositif.

Le document US 2013/0338680 A1 décrit un dispositif de plicature gastrique selon le préambule de la revendication 1.

Il ressort de ce qui précède, que la succession de ces manipulations sur un fil sont complexes, longues et fastidieuses

### Exposé de l'invention

L'un des buts de l'invention est donc de remédier aux inconvénients de l'art antérieur en proposant un dispositif de plicature gastrique par voie endoscopique, de conception simple, et qui permette de réaliser l'intervention chirurgicale d'une manière moins risquée pour la paroi de l'estomac, et plus répétable.

A cet effet, il a été mis au point un dispositif de plicature gastrique par voie endoscopique conforme à celui de l'état de la technique en ce qu'il comprend un ensemble apte à coopérer avec une poignée assujettie à des moyens de commande pour l'actionnement de l'ensemble pour la mise en place et la fixation d'une agrafe autour d'un pli formé dans une paroi gastrique.

A cet effet, l'ensemble comprend des moyens de préhension/traction d'une partie de la paroi gastrique, agencés à l'extrémité d'une tige de manipulation destinée à être reliée aux moyens de commande et actionnables pour venir s'accrocher à la paroi gastrique et effectuer une traction sur celle-ci pour former le pli.

Selon l'invention, le dispositif comprend :
- des moyens de guidage de l'agrafe autour du pli de la paroi gastrique ;
- au moins une agrafe, et des moyens de poussée de ladite agrafe dans les moyens de guidage, laquelle agrafe est destinée à venir traverser et faire une boucle autour du pli, guidée par les moyens de guidage.

De cette manière, le dispositif selon l'invention permet de réaliser une opération chirurgicale de plicature gastrique, en passant par la voie endoscopique, tout en permettant de guider une agrafe d'une manière optimale et répétable, et de fixer fermement celle-ci.

Ainsi, l'opération est réalisée en positionnant une pluralité d'agrafes successives, par exemple selon une ligne pour réduire le volume de l'estomac. Le ou les plis formés se retrouvent fermés au niveau de la paroi externe de l'estomac, avec une ou des agrafes positionnées à l'intérieur. Le volume de l'estomac peut être réduit sans problème d'étanchéité. L'opération ne dure que quelques secondes pour chaque agrafe, tandis que dans l'art antérieur, l'opération dure quelques minutes pour chaque point de suture.

Les moyens de préhension/traction sont de tout type approprié, et se présentent par exemple sous la forme d'une aiguille hélicoïdale s'étendant dans le prolongement de la tige de manipulation, laquelle est destinée à venir s'ancrer, par rotation, dans la paroi de l'estomac. Selon un autre mode de réalisation, les moyens de préhension/traction se présentent sous la forme d'un système d'aspiration.

Selon un mode de réalisation préféré, et afin d'éviter les risques d'hémorragie, les moyens de préhension/traction se présentent sous la forme d'une pince actionnable pour venir pincer et tirer sur une partie de la paroi intérieure de l'estomac en vue de former un pli.

Selon une forme de réalisation préférée, les moyens de guidage comprennent un tube de guidage débouchant sur deux mors présentant des sections en forme de gouttière ou de goulotte pour le passage et le guidage de l'agrafe. Les mors sont aptes à passer, par exemple à la manière d'une pince et sous l'action des moyens de commande, d'une position fermée de guidage dans laquelle les mors pincent le pli de la paroi gastrique, à une position ouverte de libération de l'agrafe.

Dans cette configuration, l'un des mors des moyens de guidage est par exemple fixe, tandis que l'autre mors est articulé, par exemple par l'intermédiaire d'un câble disposé le long du tube de guidage.

L'agrafe peut être de tout type approprié, notamment longiligne, et dans une forme de réalisation préférée, l'agrafe comprend une base annulaire de laquelle s'étend une tige crantée destinée à former une boucle après avoir percé et traversé la paroi de l'estomac, et à venir s'insérer et se verrouiller par encliquetage au travers de la base annulaire.

À cet effet, les moyens de poussée se présentent par exemple sous la forme d'un tube adapté pour s'insérer dans le tube de guidage, de préférence aligné à la tige de manipulation, ledit tube de poussée présentant, à une extrémité, un socle de réception de la base annulaire de l'agrafe.

Ainsi, l'agrafe et le tube de poussée sont destinés à être insérés dans le tube de guidage, et les moyens de poussée permettent de pousser l'agrafe jusque dans les mors de guidage permettant à ladite agrafe, lorsque ceux-ci pincent la paroi gastrique, de traverser la paroi gastrique et de former une boucle pour venir se verrouiller au travers de la base annulaire.

Avantageusement, l'ensemble comprend des moyens de traction de l'extrémité bouclée de l'agrafe pour la serrer et la fixer fermement.

Par exemple, les moyens de traction comprennent une tige de traction, insérée dans le tube de poussée, et présentant un ergot d'extrémité destiné à s'engager avec la tige crantée pour permettre d'effectuer une traction sur l'extrémité de ladite agrafe pour serrer la boucle.

De préférence, la tige de traction est reliée à une poignée actionnable à l'encontre de moyens élastiques de rappel pour réaliser une traction sur la tige de traction.

Avantageusement, le dispositif comprend un organe de coupe de l'extrémité de l'agrafe, monté pivotant à l'intérieur du tube de poussée, pour passer d'une position de repos à une position de coupe, et une came reliée à une poignée actionnable pour réaliser une traction sur la came, la came étant déplaçable pour forcer le passage de l'organe de coupe dans la position de coupe.

Le guidage du dispositif par voie endoscopique est réalisé, par exemple, par un endoscope comprenant une tête de vision fixée aux moyens de guidage.

### Brève description des dessins

D'autres avantages et caractéristiques ressortiront mieux de la description qui va suivre d'un mode de réalisation préféré de l'invention, donné à titre d'exemple non limitatif, en référence aux figures annexées sur lesquelles :
[Fig. 1] est une représentation schématique en perspective illustrant les moyens de guidage de l'agrafe, sous la forme de mors, et les moyens de préhension/traction sous la forme d'une pince, rapprochés rapproché d'une paroi gastrique.
[Fig. 2] est une représentation schématique similaire à celle de la figure 1, les moyens préhension/traction étant tirés en arrière pour former un pli de la paroi gastrique, et les mors étant représentés dans une position fermée de guidage, dans laquelle ils pincent le pli de la paroi gastrique.
[Fig. 3] est une représentation similaire à celle de la figure 2, une agrafe ayant été poussée vers les mors de guidage pour traverser et former une boucle autour du pli de la paroi gastrique.
[Fig. 4] une représentation similaire à celle de la figure 3, les mors des moyens de guidage étant représentés en position ouverte de libération de l'agrafe.
[Fig. 5] illustre uniquement l'agrafe, bouclée et verrouillée autour du pli de la paroi gastrique.
[Fig. 6] on représente en perspective les moyens de guidage, sous la forme de mors, en position fermée.
[Fig. 7] est une représentation similaire à celle de la figure 6, illustrant en coupe les moyens de guidage en position ouverte.
[Fig. 8] est une représentation schématique illustrant une agrafe et, en coupe, les moyens de poussée, de traction et de coupe de l'agrafe.
[Fig. 9] est une représentation de détail et en coupe des et les moyens de poussée, de traction et de coupe de l'agrafe.
[Fig. 10] est une représentation similaire à celle de la figure 9, l'agrafe ayant été poussée pour former une boucle.
[Fig. 11] est une représentation illustrant les moyens de commande, vue en perspective et en coupe.
[Fig. 12] est une représentation similaire à celle de la figure 11, vue d'un côté opposé.
[Fig. 13] est une représentation illustrant poignée de commande de la tige de traction et de l'organe de coupe.

### Description détaillée de l'invention

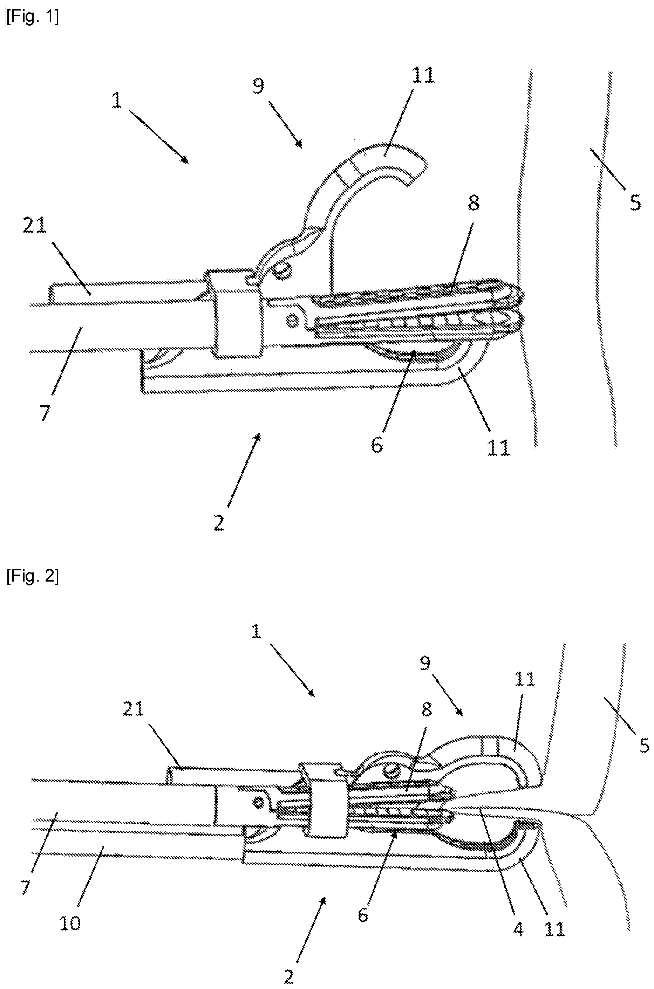

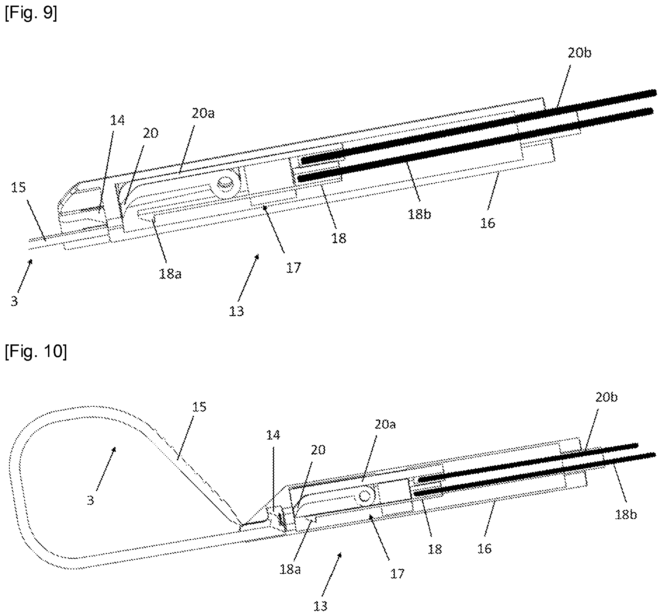

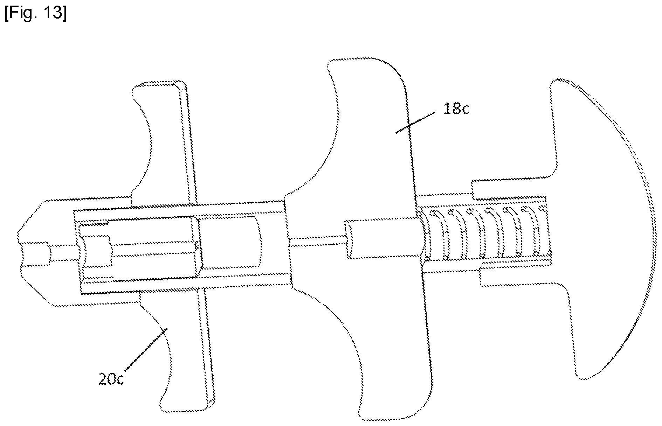

En référence aux figures 1 à 13, l'invention concerne un dispositif (1) de plicature gastrique par voie endoscopique utilisé pour le traitement de l'obésité. Le dispositif (1) est notamment destiné à venir pincer une partie de la paroi interne de l'estomac et à effectuer une traction sur celle-ci pour former un pli et fixer une agrafe autour dudit pli. Des opérations successives similaires permettent de positionner une pluralité de agrafes pour réduire le volume de l'estomac et combattre l'obésité.

À cet effet, le dispositif (1) selon l'invention est destiné à être utilisé par voie endoscopique et donc à être inséré dans un estomac par l'œsophage. Le dispositif (1) comprend un ensemble (2) apte à coopérer, d'une manière connue pour ce type de dispositif (1) utilisé par voie endoscopique, avec des moyens de commande (23) pour l'actionnement de l'ensemble (2).

L'ensemble (2) du dispositif (1) permet ainsi, sous l'action des moyens de commande (23), la mise en place et à la fixation d'une agrafe (3) autour d'un pli (4) formé dans une paroi gastrique (5).

À cet effet, l'ensemble (2) comprend des moyens de préhension/traction (6) d'une partie de la paroi gastrique (5), agencés à l'extrémité d'une tige de manipulation (7) destinée à être reliée aux moyens de commande (23) et actionnables par lesdits moyens de commande (23) pour venir s'accrocher à la paroi gastrique (5) et effectuer une traction sur celle-ci pour former le pli (4).

Ces moyens de préhension/traction (6) peuvent être de tout type approprié, tel que par exemple sous la forme d'une aiguille hélicoïdale destinée à venir se planter dans la paroi gastrique (5), ou bien sous la forme d'un système d'aspiration, mais d'une manière préférée, les moyens de préhension/traction (6) se présentent sous la forme d'une pince (8) articulée.

De cette manière, une fois insérée dans l'estomac, un chirurgien peut, en actionnant les moyens de commande (23), manipuler la pince (8) pour venir pincer la paroi gastrique (5), et former un pli (4) en tirant sur la pince (8).

La pince (8) comprend par exemple un mors fixe et un mors articulé, par exemple à l'encontre d'un organe élastique de rappel qui tend à plaquer le mors articulé contre le mors fixe dans une position de pincement. L'articulation du mors articulé peut se faire par exemple par traction sur un câble fixé, d'une part au mors articulé et, d'autre part relié aux moyens de commande (23) en passant, par exemple, à l'intérieur de la tige de manipulation (7). Les mors des moyens de préhension/traction (6) sont par exemple plats et présentent une surface de contact crantée pour s'agripper d'une manière optimale à la paroi gastrique (5).

L'ensemble (2) du dispositif (1) selon l'invention comprend également des moyens de guidage (9) d'une agrafe (3) autour du pli (4) de la paroi gastrique (5). Ces moyens de guidage (9) sont destinés à guider une agrafe (3), poussée au travers eux, afin que ladite agrafe (3) fasse une boucle autour du pli (4) de la paroi gastrique (5).

À cet effet, les moyens de guidage (9) comprennent, par exemple, un tube de guidage (10) débouchant sur deux mors (11) présentant des sections en forme de gouttières, notamment ouvertes en regard l'une de l'autre, pour le passage et le guidage de l'agrafe (3). Les mors (11) sont aptes à passer, sous l'action des moyens de commande (23), d'une position fermée de guidage dans laquelle les mors (11) pincent le pli (4) de la paroi gastrique (5) et viennent en regard l'un de l'autre pour former un chemin pour l'agrafe (3), à une position ouverte de libération de l'agrafe (3).

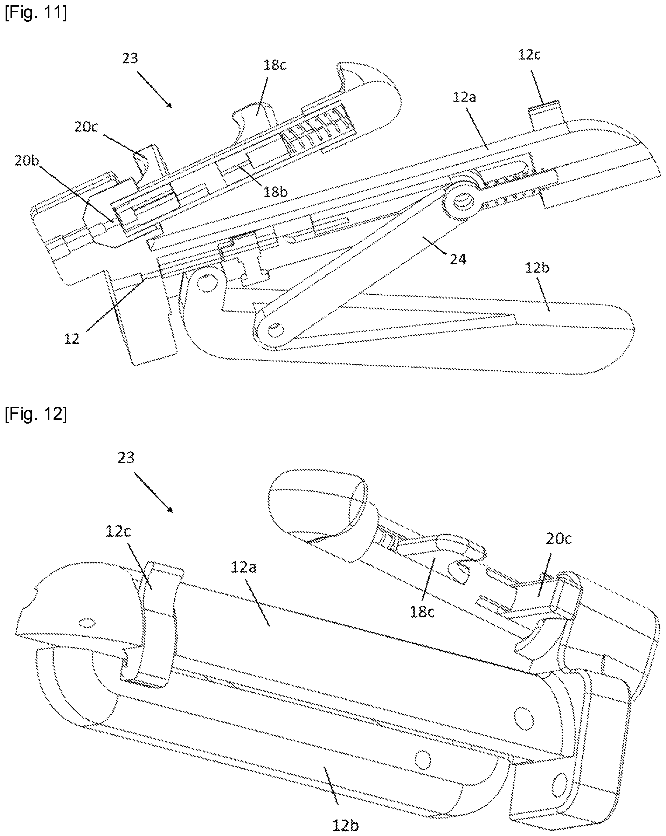

Les mors (11) de moyens de guidage (9) sont articulés de toute manière appropriée, et de préférence de la même manière que les mors de la pince (8). Par exemple, l'un des mors (11) des moyens de guidage (9) est fixe, tandis que l'autre est articulé par l'intermédiaire d'un câble (12). En référence aux figures 11 et 12 ; le câble (12) est par exemple relié aux moyens de commande (23), qui comprennent deux portions (12a, 12b) montées pivotantes l'une par rapport à l'autre entre une position ouverte, figure 11, correspondant à une position de non-traction sur le câble (12) et une position ouverte des mors (11), à une position fermée, figure 12, correspondant à une position de traction sur le câble (12) et de fermeture des mors (11). La position fermée est par exemple verrouillée par au moins un loquet (12c) monté sur l'une des portions (12a, 12b) et venant d'engager dans l'autre portion (12b, 12a).

La traction sur le câble (12) est par exemple réalisée par un système de bielle (24) comprenant une première extrémité montée pivotante avec l'une des portions (12b), tandis que la deuxième extrémité est montée pivotante et coulissante à l'encontre d'un ressort à l'intérieur de l'autre portion (12a). Ladite deuxième extrémité est rattachée au câble (12) pour y exercer une traction lorsque les deux portions (12a, 12b) sont rapprochées l'une de l'autre.

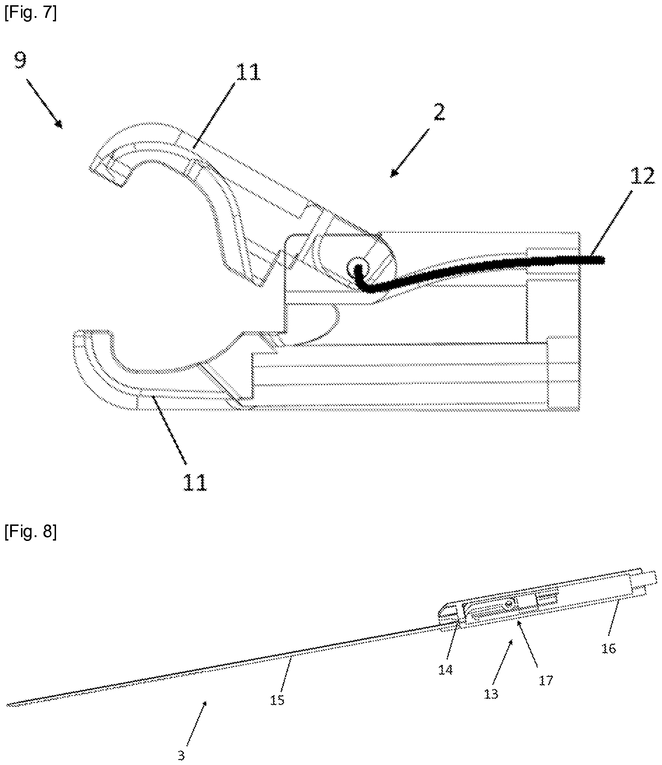

En référence aux figures 8 à 10, l'ensemble (2) du dispositif (1) de plicature selon l'invention comprend également une agrafe (3) et des moyens de poussée (13) de ladite agrafe (3) dans les moyens de guidage (9), ladite agrafe (3) étant destinée à venir traverser le pli (4) de la paroi gastrique (5) et faire une boucle autour de celui-ci, en étant guidée par les moyens de guidage (9).

L'agrafe (3) présente toute forme appropriée et comprend une base annulaire (14) de laquelle s'étend une tige crantée (15) destinée à boucler et à venir s'insérer et se verrouiller par encliquetage au travers de la base annulaire (14).

Les moyens de poussée (13) de l'agrafe (3) se présentent, par exemple, sous la forme d'un tube de poussée (16) adapté pour s'insérer dans le tube de guidage (10). Ce tube de poussée (16) présente, à une extrémité, un socle de réception de la base annulaire (14) de l'agrafe (3).

Ainsi, l'agrafe (3) peut être positionnée à l'extrémité du tube de poussée (16), et dans le prolongement de celui-ci. L'agrafe (3) et le tube de poussée (16) sont destinés à être insérés dans le tube de guidage (10) et, par poussée sur le tube de poussée (16), cela permet de guider l'agrafe (3) dans les moyens de guidage (9), dont les mors (11) en position fermée forment une sorte de rampe pour faire boucler l'agrafe (3) de sorte que l'extrémité libre de l'agrafe (3) vienne s'insérer et se verrouiller par encliquetage au travers de la base annulaire (14), en pénétrant notamment à l'intérieur du tube de poussée (16).

Afin de serrer la boucle formée par l'agrafe (3), l'ensemble (2) du dispositif (1) selon l'invention comprend des moyens de traction (17) de l'extrémité bouclée de l'agrafe (3).

Par exemple, les moyens de traction (17) comprennent une tige de traction (18) insérée dans le tube de poussée (16) et présentant un ergot d'extrémité (18a) destiné à s'engager avec la tige crantée (15) pour permettre d'effectuer une traction sur l'extrémité de l'agrafe (3) et serrer la boucle. La traction est par exemple réalisée par l'intermédiaire d'un câble (18b) reliée à une poignée (18c) que comprend éventuellement les moyens de commande (23), actionnable à l'encontre de moyens élastiques de rappel tels qu'un ressort, voir figure 11.

Une fois serrée, l'extrémité libre et bouclée de l'agrafe (3) nécessite d'être coupée pour ne pas gêner ou blesser l'intérieur de l'estomac. À cet effet, le tube de poussée (16), comprend un organe de coupe (20) de l'extrémité de l'agrafe (3), monté pivotant à l'intérieur du tube de poussée (16), pour passer d'une position de repos à une position de coupe.

Le tube de poussé (16) comprend une came (20a) reliée, par exemple par un câble (20b) à une poignée (20c), que comprend éventuellement les moyens de commande (23), actionnable pour réaliser une traction sur la came (20b), la came étant déplaçable pour forcer le passage de l'organe de coupe (20) dans la position de coupe.

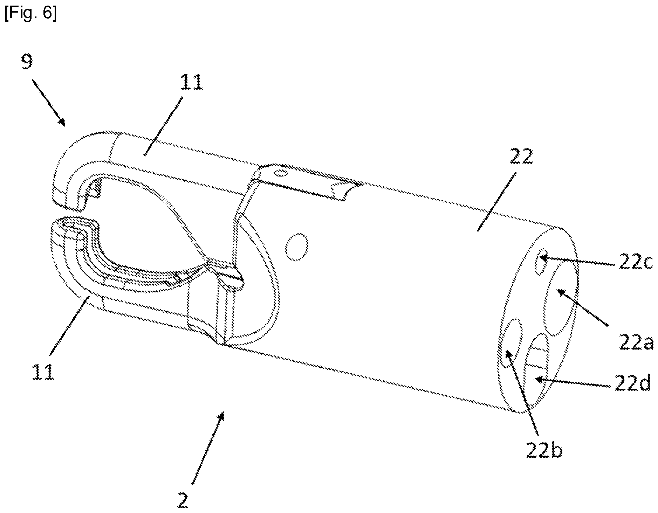

Afin de guider le dispositif (1) selon sur l'invention à l'intérieur de l'estomac d'un patient, l'ensemble (2) comprend un endoscope comprenant une tête de vision (21) fixée aux moyens de guidage (9). Par exemple, et en référence à la figure 6, les moyens de guidage (9) comprennent une embase (22) présentant un alésage (22a) pour le passage de l'endoscope (21), un alésage (22b) pour le passage de la pince (8), un alésage (22c) pour le passage du câble (12) de fermetures des mors (11), un alésage (22d) pour le passage de l'agrafe (3) et du tube de poussée (16).

Il est maintenant décrit le procédé d'utilisation du dispositif (1) de plicature gastrique selon l'invention.

Le dispositif (1) est inséré dans l'œsophage d'un patient jusqu'à pénétrer dans l'estomac.

Les moyens de préhension/traction (6), et les moyens de guidage (9) sont en position ouverte et le dispositif (1) est approché de la paroi gastrique (5) jusqu'à y venir en contact.

En référence à la figure 1, la pince (8) des moyens de préhension/traction (6) est refermée pour saisir une partie de la paroi gastrique (5) et est tirée en arrière pour former un pli (4).

En référence à la figure 2, les mors (11) des moyens de guidage (9) sont refermés pour pincer le pli (4) de la paroi gastrique (5).

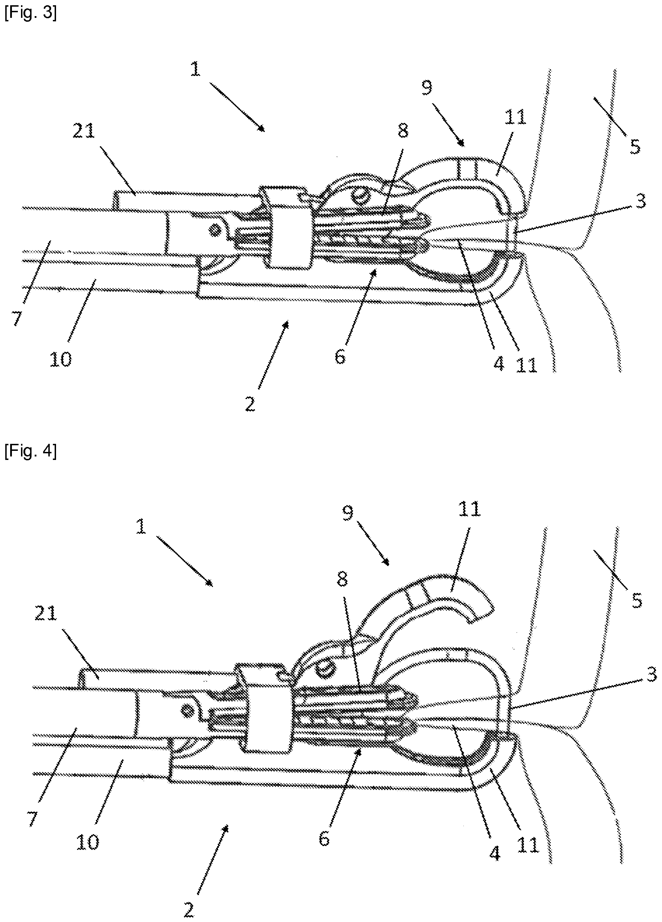

En référence à la figure 3, une agrafe (3) insérée dans le tube de guidage (10) est poussée vers lesdits moyens de guidage (9) par le tube de poussée (16).

L'agrafe (3), guidée par la forme en gouttière ou goulotte des mors (11) des moyens de guidage (9) transperce et traverse le pli (4) gastrique et vient former une boucle jusqu'à ce que l'extrémité libre de l'agrafe (3) s'engage à l'intérieur de la base annulaire (14) de l'agrafe (3) et s'insère à l'intérieur du tube de poussée (16).

L'insertion de l'extrémité libre de l'agrafe (3) dans la base annulaire (14) permet le verrouillage par encliquetage des crans de ladite agrafe (3) avec la base annulaire (14) de l'agrafe (3), et avec l'ergot (18a) de la tige de traction (18).

Ensuite, l'extrémité libre de l'agrafe (3) insérée à l'intérieur du tube de poussée (16), et engagée avec l'ergot (18a) de la tige de traction (18), est serrée en effectuant une traction sur ladite tige de traction (18). Ensuite, la partie superflue de l'agrafe (3) est coupée traction sur la came (20a) pour faire pivoter l'organe de coupe (20).

Ensuite, en référence à la figure 4, les mors (11) des moyens de guidage (9) sont retournés en position ouverte de libération. En effet, dans cette position, les mors (11) des moyens de guidage (9) peuvent être retirés en libérant l'agrafe (3).

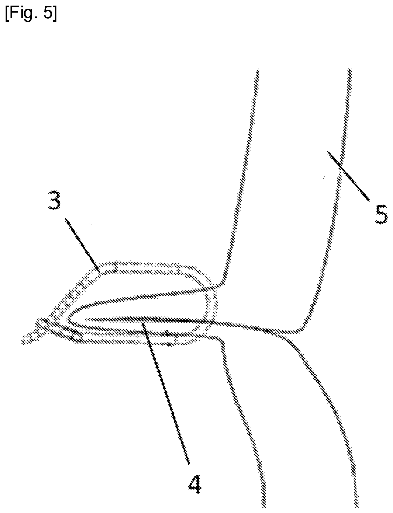

La pince (8) des moyens de préhension/traction (6) est également ouverte, pour permettre le retrait du dispositif (1) et laisser en place l'agrafe (3), voir figure 5.

Ces étapes successives sont réalisées autant de fois que nécessaire pour positionner une pluralité de agrafes (3) autour de plis (4) formés à l'intérieur de la paroi gastrique (5) pour en réduire le volume de l'estomac et combattre l'obésité.

## Revendications

1. Dispositif (1) de plicature gastrique par voie endoscopique comprenant un ensemble (2) apte à coopérer avec une poignée assujettie à des moyens de commande (23) pour l'actionnement de l'ensemble (2) pour la mise en place et la fixation d'une agrafe (3) autour d'un pli (4) d'une paroi gastrique (5), comprenant:
- des moyens de préhension/traction (6) d'une partie d'une paroi gastrique (5), agencés à l'extrémité d'une tige de manipulation (7) destinée à être reliée aux moyens de commande (23) et actionnables pour venir s'accrocher à la paroi gastrique (5) et effectuer une traction sur celle-ci pour former un pli (4) ;
- des moyens de guidage (9) d'une agrafe (3) autour du pli (4) de la paroi gastrique (5) ;
- au moins une agrafe (3) et des moyens de poussée (13) de l'agrafe (3) dans les moyens de guidage (9), laquelle agrafe (3) est destinée à venir traverser et faire une boucle autour du pli (4), guidée par les moyens de guidage (9) ;
**caractérisé en ce qu'**il comprend :
- des moyens de traction (17) de l'extrémité bouclée de l'agrafe (3).

2. Dispositif (1) selon la revendication 1, ***caractérisé* en ce que** les moyens de préhension/traction (6) se présentent sous la forme d'une pince (8).

3. Dispositif (1) selon la revendication 1, ***caractérisé* en ce que** les moyens de préhension/traction (6) se présentent sous la forme d'une aiguille hélicoïdale s'étendant dans le prolongement de la tige de manipulation (7).

4. Dispositif (1) selon la revendication 1, ***caractérisé* en ce que** les moyens de préhension/traction (6) se présentent sous la forme d'un système d'aspiration.

5. Dispositif (1) selon la revendication 1, ***caractérisé* en ce que** les moyens de guidage (9) comprennent un tube de guidage (10) débouchant sur deux mors (11) articulés présentant des sections en forme de gouttière pour le passage et le guidage de l'agrafe (3), les mors (11) étant aptes à passer, sous l'action des moyens de commande (23), d'une position fermée de guidage, dans laquelle les mors (11) pincent le pli (4) de la paroi gastrique (5), à une position ouverte de libération de l'agrafe (3).

6. Dispositif (1) selon la revendication 1, ***caractérisé* en ce que** l'agrafe (3) comprend une base annulaire (14) et une tige crantée (15), la tige crantée (15) étant destinée à boucler et à venir s'insérer et se verrouiller par encliquetage au travers de la base annulaire (14).

7. Dispositif (1) selon la revendication 6, ***caractérisé* en ce que** les moyens de poussée (13) se présentent sous la forme d'un tube de poussée (16), adapté pour s'insérer dans le tube de guidage (10), et présentant à une extrémité un socle de réception de la base annulaire (14) de l'agrafe (3).

8. Dispositif (1) selon la revendication 7, ***caractérisé* en ce que** les moyens de traction (17) comprennent une tige de traction (18), insérée dans le tube de poussée (16), la tige de traction (18) présentant un ergot d'extrémité (18a) destiné à s'engager avec la tige crantée (15) pour permettre d'effectuer une traction sur l'extrémité de l'agrafe (3) pour serrer la boucle.

9. Dispositif (1) selon la revendication 7, ***caractérisé* en ce que** la tige de traction (18) est reliée à une poignée (18c) actionnable à l'encontre de moyens élastiques de rappel pour réaliser une traction sur la tige de traction.

10. Dispositif (1) selon la revendication 7, ***caractérisé* en ce qu'**il comprend un organe de coupe de l'extrémité de l'agrafe (3), monté pivotant à l'intérieur du tube de poussée, pour passer d'une position de repos à une position de coupe, et une came reliée à une poignée actionnable pour réaliser une traction sur la came, la came étant déplaçable pour forcer le passage de l'organe de coupe dans la position de coupe.

11. Dispositif (1) selon la revendication 1, ***caractérisé* en ce que** le dispositif (1) comprend un endoscope comprenant une tête de vision (21) fixée aux moyens de guidage (9).

## Patentansprüche

1. Ein endoskopisches Magenfaltenvorrichtung (1), umfassend eine Baugruppe (2), die in der Lage ist, mit einem Griff zu kooperieren, der an Steuerungsmittel (23) befestigt ist, um die Baugruppe (2) zu betätigen, um eine Klammer (3) um eine Falte (4) einer Magenwand (5) zu positionieren und zu fixieren, umfassend:
- Mittel (6) zum Greifen/Ziehen eines Teils einer Magenwand (5), die am Ende eines Manipulationsschafts (7) angeordnet sind, der dazu bestimmt ist, mit den Steuerungsmitteln (23) verbunden zu werden und die betätigt werden können, um in Eingriff mit der Magenwand (5) zu kommen und daran zu ziehen, um eine Falte (4) zu bilden;
- Mittel (9) zum Führen einer Klammer (3) um die Falte (4) der Magenwand (5);
- mindestens eine Klammer (3) und Mittel (13) zum Schieben der Klammer (3) in die Führungsvorrichtung (9), wobei die Klammer (3) dazu bestimmt ist, durch die Falte (4) geführt zu werden und eine Schleife um die Falte (4) zu bilden, geführt durch die Führungsvorrichtung (9);
**dadurch gekennzeichnet, dass** es umfasst:
- Mittel (17) zum Ziehen des geschleiften Endes der Klammer (3).

2. Die Vorrichtung (1) nach Anspruch 1, **dadurch gekennzeichnet, dass** die Greif-/Ziehvorrichtung (6) in Form einer Klemme (8) vorliegt.

3. Die Vorrichtung (1) nach Anspruch 1, **dadurch gekennzeichnet, dass** die Greif-/Ziehvorrichtung (6) in Form einer Helixnadel vorliegt, die in der Verlängerung des Manipulationsschafts (7) verläuft.

4. Die Vorrichtung (1) nach Anspruch 1, **dadurch gekennzeichnet, dass** die Greif-/Ziehvorrichtung (6) in Form eines Vakuumsystems vorliegt.

5. Die Vorrichtung (1) nach Anspruch 1, **dadurch gekennzeichnet, dass** die Führungsvorrichtung (9) ein Führungsschlauch (10) umfasst, der in zwei gelenkige Backen (11) mündet, die Abschnitte in Form einer Rinne zum Durchführen und Führen der Klammer (3) aufweisen, wobei die Backen (11) in der Lage sind, sich unter der Wirkung der Steuerungsmittel (23) von einer geschlossenen Führungsposition, in der die Backen (11) die Falte (4) der Magenwand (5) klemmen, in eine offene Position zum Freigeben der Klammer (3) zu bewegen.

6. Die Vorrichtung (1) nach Anspruch 1, **dadurch gekennzeichnet, dass** die Klammer (3) eine ringförmige Basis (14) und einen gezahnten Schaft (15) umfasst, wobei der gezahnte Schaft (15) dazu bestimmt ist, geschleift zu werden und in die ringförmige Basis (14) eingeführt und durch Einrasten verriegelt zu werden.

7. Die Vorrichtung (1) nach Anspruch 6, **dadurch gekennzeichnet, dass** die Schiebemittel (13) in Form eines Schiebeschlauchs (16) vorliegen, der dazu angepasst ist, in den Führungsschlauch (10) eingeführt zu werden und an einem Ende eine Andockstelle zum Aufnehmen der ringförmigen Basis (14) der Klammer (3) aufweist.

8. Die Vorrichtung (1) nach Anspruch 7, **dadurch gekennzeichnet, dass** die Ziehvorrichtung (17) eine Ziehwelle (18) umfasst, die in den Schiebeschlauch (16) eingeführt ist, wobei die Ziehwelle (18) einen Endzapfen (18a) aufweist, der dazu bestimmt ist, mit dem gezahnten Schaft (15) in Eingriff zu kommen, um das Ende der Klammer (3) zu ziehen und die Schleife zu spannen.

9. Die Vorrichtung (1) nach Anspruch 7, **dadurch gekennzeichnet, dass** die Ziehwelle (18) mit einem Griff (18c) verbunden ist, der gegen elastische Rückstellmittel betätigt werden kann, um einen Zug auf die Ziehwelle zu erzeugen.

10. Die Vorrichtung (1) nach Anspruch 7, **dadurch gekennzeichnet, dass** sie ein Element zum Schneiden des Endes der Klammer (3) umfasst, das schwenkbar im Schiebeschlauch montiert ist, um von einer Ruheposition in eine Schneidposition überzugehen, und eine Nocke, die mit einem Griff verbunden ist, der betätigt werden kann, um an der Nocke zu ziehen, wobei die Nocke verschiebbar ist, um das Schneidelement zu zwingen, in seine Schneidposition zu gelangen.

11. Die Vorrichtung (1) nach Anspruch 1, **dadurch gekennzeichnet, dass** die Vorrichtung (1) ein Endoskop umfasst, das einen Sichtkopf (21) umfasst, der an der Führungsvorrichtung (9) befestigt ist.

## Claims

1. An endoscopic gastric plication device (1) comprising an assembly (2) which is capable of cooperating with a handle secured to control means (23) in order to actuate the assembly (2) for positioning and fixing a staple (3) around a fold (4) of a gastric wall (5), comprising:
- means (6) for grasping/pulling a portion of a gastric wall (5) arranged at the end of a manipulating shaft (7) intended to be connected to the control means (23) and which can be actuated in order to come into engagement with the gastric wall (5) and pull on it in order to form a fold (4);
- means (9) for guiding a staple (3) around the fold (4) of the gastric wall (5);
- at least one staple (3), and means (13) for pushing the staple (3) in the guiding means (9), that staple (3) being intended to come to be passed through and make a loop around the fold (4), guided by the guiding means (9);
***characterized in that*** it comprises:
- means (17) for pulling the looped end of the staple (3).

2. The device (1) as claimed in claim 1, ***characterized in that*** the grasping/pulling means (6) are in the form of a clamp (8).

3. The device (1) as claimed in claim 1, ***characterized in that*** the grasping/pulling means (6) are in the form of a helical needle extending in the extension of the manipulating shaft (7).

4. The device (1) as claimed in claim 1, ***characterized in that*** the grasping/pulling means (6) are in the form of a vacuum system.

5. The device (1) as claimed in claim 1, ***characterized in that*** the guiding means (9) comprise a guiding tube (10) opening onto two articulated jaws (11) having sections in the form of a trough for passing the staple (3) through and guiding it, the jaws (11) being capable of moving, under the action of the control means (23), from a closed guiding position in which the jaws (11) clamp the fold (4) of the gastric wall (5), to an open position for releasing the staple (3).

6. The device (1) as claimed in claim 1, ***characterized in that*** the staple (3) comprises an annular base (14) and a notched shaft (15), the notched shaft (15) being intended to be looped and to come to be inserted into and be locked by snap fitting through the annular base (14).

7. The device (1) as claimed in claim 6, ***characterized in that*** the pushing means (13) are in the form of a pushing tube (16) which is adapted to be inserted in the guiding tube (10) and has a dock at one end for receiving the annular base (14) of the staple (3).

8. The device (1) as claimed in claim 7, ***characterized in that*** the pulling means (17) comprise a pulling shaft (18) inserted in the pushing tube (16), the pulling shaft (18) having an end lug (18a) intended to engage with the notched shaft (15) in order to be able to pull on the end of the staple (3) to tighten the loop.

9. The device (1) as claimed in claim 7, ***characterized in that*** the pulling shaft (18) is connected to a handle (18c) which can be actuated against elastic restoring means in order to produce a pull on the pulling shaft.

10. The device (1) as claimed in claim 7, ***characterized in that*** it comprises a member for cutting the end of the staple (3), pivotably mounted inside the pushing tube in order to pass from a rest position to a cutting position, and a cam connected to a handle which can be actuated in order to pull on the cam, the cam being displaceable in order to force the cutting member to move into its cutting position.

11. The device (1) as claimed in claim 1, ***characterized in that*** the device (1) comprises an endoscope comprising a viewing head (21) fixed to the guiding means (9).
